# EUROPEAN PATENT APPLICATION

(11) **EP 2 783 632 A1**
(43) Date of publication of application: **01.10.2014**
(21) Application number: 14161838.9
(22) Date of filing: 26.03.2014
(51) Int. Cl.: A61B 6/00, G06F 19/00, G06F 3/14, A61B 8/00

(54) **Mobile terminal apparatus having radio communication interface**

(30) Priority: 26.03.2013 JP 2013064661
(71) Applicant: FUJIFILM Corporation, Minato-ku Tokyo 106-8620 (JP)
(72) Inventor: Kudo, Yuya, Tokyo, 106-8620 (JP); Ueda, Satoshi, Tokyo, 106-8620 (JP); Okusu, Kiyohisa, Tokyo, 106-8620 (JP); Matsumata, Hironori, Tokyo, 106-8620 (JP); Ohta, Yasunori, Kanagawa (JP)
(74) Representative: Klunker . Schmitt-Nilson . Hirsch

(57) **Abstract**

A mobile terminal apparatus having a radio communication interface (28) for radio communication includes a touchscreen device (22). A program receiver (51) receives program (CAP) from a server apparatus (16) storing plural sets of program, for communication access to an imaging apparatus (13A-13C). A controller (50) functions according to the received program, drives the touchscreen device to display a user interface view (53, 55, 55A-55C, 59), and causes the radio communication interface to perform the communication access to the imaging apparatus. Preferably, the controller transmits a signal to and receives a signal from the imaging apparatus by the communication access. Furthermore, the touchscreen device as an information input device inputs modality information for recognition of the imaging apparatus. The program receiver transmits the input modality information to the server apparatus, and acquires the program according to the modality information from the server apparatus.

## Description

### BACKGROUND OF THE INVENTION

### 1. Field of the Invention

The present invention relates to a mobile terminal apparatus having a radio communication interface. More particularly, the present invention relates to a mobile terminal apparatus which has a radio communication interface, of which communication access to a plurality of modalities is possible, and of which an application program can be readily updated.

### 2. Description Related to the Prior Art

Various modalities as diagnostic imaging apparatuses are known in the field of medicine, such as an X-ray imaging apparatus, an endoscope (endoscope apparatus) and the like. A terminal apparatus of a console configuration is provided for controlling the modality by transmitting a control signal for imaging and the like. A cable is used for connecting the terminal apparatus to the modality to send the control signal to the modality. It is likely that plural modalities of a common imaging method are used at the same time. The terminal apparatus is prepared for each one of the modalities, for example, each one of the plural endoscopes. The number of the terminal apparatuses increases according to an increase in the number of the modalities. There occur a problem in requiring a large space for disposing all of the terminal apparatuses and complicated handling for selecting the terminal apparatuses for respectively the modalities.

JP-A 2007-175229 discloses a type of the terminal apparatus which wirelessly communicates for transmitting the control signal, and is capable to control the plural endoscopes. The terminal apparatus for medical use is different from a remote controller for a television set having only a transmitting function for transmitting the control signal to the television set. The terminal apparatus has the transmitting function, a receiving function and a display function. The transmitting function transmits the control signal to the modality. The receiving function receives a diagnostic image, various data and the like from the modality. The display function displays the received image and data. While an operator carries the terminal apparatus as a single device, communication access to the plural endoscopes is possible without use of plural terminal apparatuses. Thus, there is no laborious operation for the operator to utilize the plural terminal apparatuses at one time distinctly.

It is possible according to JP-A 2007-175229 to carry out the communication access to plural modalities of the common the imaging method, for example, plural endoscopes. Furthermore, it is likely that the endoscope, the X-ray imaging apparatus and the like are used in one hospital facility, as the modalities of two or more imaging methods. Also, a plurality of types of the endoscopes may be used, with a difference in the specifics or the like. A problem may arise in impossibility of the communication access to the endoscopes with the terminal apparatus due to differences in the control signal.

For the communication access of the modalities of the imaging methods by use of the terminal apparatus disclosed in JP-A 2007-175229, the terminal apparatus must be prepared for each one of the imaging methods of the modalities. Exchange of the terminal apparatuses for respectively the imaging methods of the modalities, an operator must have the plural terminal apparatuses to cause very complicated handling.

The terminal apparatus for the modality is unlike a universal remote controller with a learning function for the television set, because the terminal apparatus not only stores the control signal but requires a function for receiving an image and data from the modality. The terminal apparatus for the modality of a known structure is not compatible to imaging methods in the manner similar to the universal remote controller with the learning function for the television set.

To solve such problems, it is conceivable to install a remote application program CAP (program information) in the terminal apparatus for compatibility to the modalities of the imaging methods, to enable the communication access to the modalities with a difference in the imaging method.

Although the remote application program CAP is adapted to the modalities of the imaging methods, there occurs an exchange of the modalities in the hospital facility. The remote application program CAP must be updated at each time of starting the use of a new apparatus. Withdrawing the terminal apparatus from the operator for the purpose of installing the remote application program CAP requires considerable time and labor cost for updating. The number of the terminal apparatuses may be very high according to a scale of the hospital facility, because of the high number of the operators such as doctors or other medical service providers. A problem arises as long time and hard work are required for installing the remote application program CAP for all the terminal apparatuses.

There is no suggestion in JP-A 2007-175229 for the communication access of the modalities with a difference in the imaging method by use of the terminal apparatus.

### SUMMARY OF THE INVENTION

In view of the foregoing problems, an object of the present invention is to provide a mobile terminal apparatus which has a radio communication interface, of which communication access to a plurality of modalities is possible, and of which an application program can be readily updated.

In order to achieve the above and other objects and advantages of this invention, a mobile terminal apparatus having a radio communication interface for radio communication is provided. There is a display device. A program receiver receives a particular program for a particular modality of interest from a program storage apparatus storing plural programs in association with respectively modalities. A controller functions according to the particular program, drives the display device to display a user interface view, and causes the radio communication interface to perform communication access to the particular modality.

Preferably, the controller transmits a signal to and receives a signal from the particular modality by the communication access.

Preferably, furthermore, an information input device inputs modality information for recognition of each of the modalities. The program receiver transmits the input modality information to the program storage apparatus, and acquires the particular program from the program storage apparatus.

Preferably, the information input device is externally operated to input the modality information.

In another preferred embodiment, the information input device receives the modality information transmitted from the particular modality with the radio communication interface.

Preferably, the information input device is a short range radio communication interface discrete from the radio communication interface for receiving the modality information from the particular modality.

Preferably, the controller carries out display control for displaying received data on the display device upon reception from the particular modality.

Preferably, the received data is an image.

Preferably, the program receiver accesses the program storage apparatus by use of the radio communication interface.

Preferably, the modality is at least one of a CT imaging apparatus, X-ray imaging apparatus and mammography apparatus.

Preferably, the user interface view includes a request area for requesting the modality to create an image.

Preferably, the request area is used for inputting at least one of name data of a body, identification data of the body, and body part data in the body.

Preferably, the user interface view includes a condition setting area for inputting parameter data of condition setting of the modality.

Preferably, the parameter data is at least one of data of a current, voltage, speed and size.

Preferably, the user interface view includes a maintenance area for transmitting or receiving relevant data of maintenance of the modality.

Preferably, the relevant data is at least one of command data of reset, command data of calibration, use history data and error history data of the modality.

Also, a diagnosis system is provided, and includes a modality for diagnosis, a program storage apparatus for storing plural sets of program information, and a mobile terminal apparatus. The mobile terminal apparatus includes a radio communication interface for radio communication, and a display device. A program receiver receives program information from the program storage apparatus, for communication access to the modality. A controller functions according to the received program information, drives the display device to display a user interface view, and causes the radio communication interface to perform the communication access to the modality.

Also, a method of setting radio communication with a modality is provided, and includes a step of receiving program information selectively among plural sets of program information, for communication access to the modality. A user interface view is displayed according to the received program information. The communication access is performed to the modality according to the received program information.

Also, a computer executable program for setting radio communication with a modality is provided, and includes a program code for receiving program information selectively among plural sets of program information, for communication access to the modality. A program code is for displaying a user interface view according to the received program information. A program code is for performing the communication access to the modality according to the received program information.

Accordingly, communication access to a plurality of modalities from the mobile terminal apparatus is possible, and an application program can be readily updated, because the program information is received for the communication access to the modality.

### BRIEF DESCRIPTION OF THE DRAWINGS

The above objects and advantages of the present invention will become more apparent from the following detailed description when read in connection with the accompanying drawings, in which:
Fig. 1 is an explanatory view illustrating a mobile terminal apparatus;
Fig. 2 is a block diagram illustrating the mobile terminal apparatus, a hospital facility and a server apparatus;
Fig. 3 is a block diagram illustrating reception of a remote application program CAP to the mobile terminal apparatus;
Fig. 4 is a block diagram illustrating a user interface view in the mobile terminal apparatus;
Fig. 5 is a plan illustrating a menu image on the mobile terminal apparatus;
Fig. 6 is a plan illustrating a request area on the mobile terminal apparatus;
Fig. 7 is a plan illustrating a condition setting area on the mobile terminal apparatus;
Fig. 8 is a plan illustrating a viewing area on the mobile terminal apparatus;
Fig. 9 is a plan illustrating a maintenance area on the mobile terminal apparatus;
Fig. 10 is a flow chart illustrating reception of a remote application program CAP to the mobile terminal apparatus;
Fig. 11 is a block diagram illustrating a preferred embodiment with a short range radio communication interface.

### DETAILED DESCRIPTION OF THE PREFERRED EMBODIMENT(S) OF THE PRESENT INVENTION

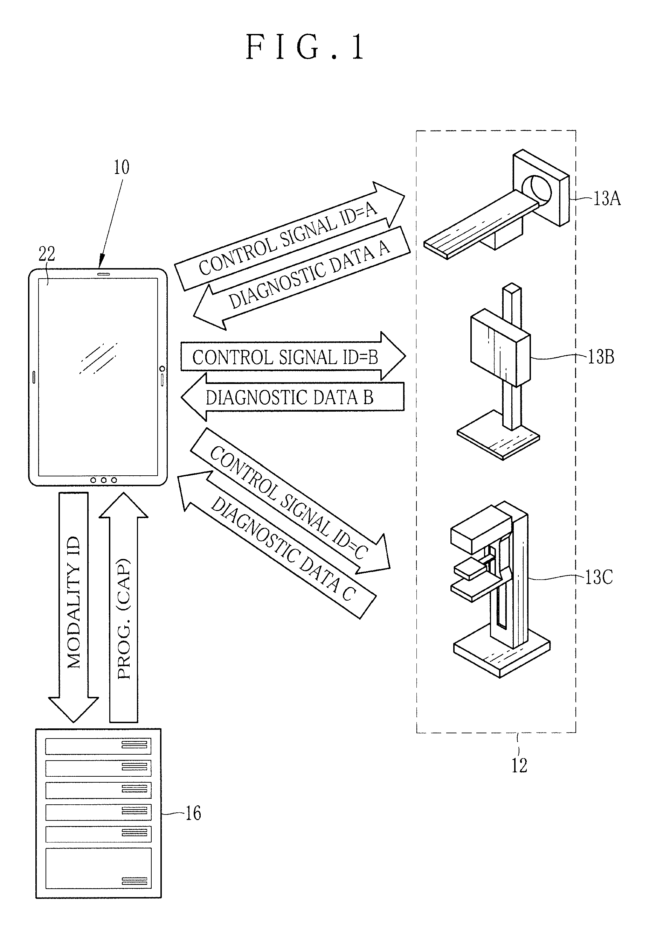

In Fig. 1, a mobile terminal apparatus 10 of a console configuration of the invention is used for communication access to various modalities installed in a hospital facility 12. The modalities include a CT imaging apparatus 13A (computed tomography apparatus) as a modality A, an X-ray imaging apparatus 13B as a modality B, and a mammography apparatus 13C as a modality C. The CT imaging apparatus 13A forms a sectional image of a body by use of X-rays. The X-ray imaging apparatus 13B forms a radiographic image of a chest, head or the like of a body by use of X-rays. The mammography apparatus 13C forms an image of breast by use of X-rays.

The mobile terminal apparatus 10 has various functions for remote management, including a transmission function for transmitting a command signal to the imaging apparatuses 13A-13C, a receiving function for receiving the image formed by the imaging apparatuses 13A-13C, a display function for displaying the image or data received from the imaging apparatuses 13A-13C, a condition setting function for setting an imaging condition of the imaging apparatuses 13A-13C. In the mobile terminal apparatus 10 is disposed a touchscreen device 22 as a display device and information input device or user interface, for displaying a user interface view, received diagnostic data and the like.

The mobile terminal apparatus 10 downloads (acquires) a remote application program CAP (program information) from a server apparatus 16 as a program storage apparatus for the remote management in association with the imaging apparatuses 13A-13C, and automatically installs the remote application program CAP.

Remote application programs CAP in association with the imaging apparatuses 13A-13C are stored in the server apparatus 16. In case the mobile terminal apparatus 10 specifies a modality ID (modality type code as modality information) for recognizing the imaging apparatuses 13A-13C and sends a request of a remote application program CAP to the server apparatus 16, then the server apparatus 16 distributes the remote application program CAP according to the specified modality ID to the mobile terminal apparatus 10. In response, the mobile terminal apparatus 10 automatically installs the remote application program CAP. Note that the information of the modality ID is constituted by a manufacturer, apparatus type, and product ID of the modality. In the present specification, the modality ID is designated with symbols of A, B and C in a simplified manner.

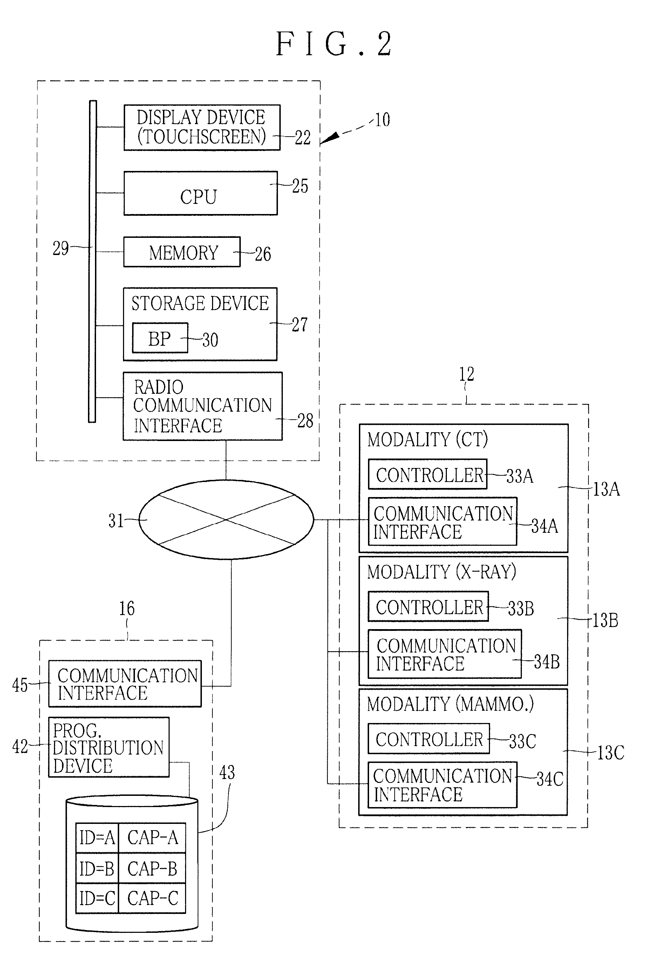

In Fig. 2, the mobile terminal apparatus 10 is constituted by an electronic equipment and computer-executable programs installed therein. Examples of the electronic equipment are a cellular telephone, smart phone and other mobile terminal apparatuses. Examples of the programs are a control program such as an operating system (OS) and a basic program 30 (BP) for running a plurality of remote application programs CAP. The mobile terminal apparatus 10 includes a quadrilateral housing of a small thickness, a CPU 25, a memory 26, a storage device 27 and a radio communication interface 28 as an information input device, which are interconnected by a data bus 29 together with the touchscreen device 22. The touchscreen device 22 is a display device for receiving manual inputs of an operator. The touchscreen device 22 is disposed on a front or rear surface of the housing.

The storage device 27 stores various data, and is a semiconductor memory such as a hard disk drive, flash memory and the like. Also, the storage device 27 stores the control program, the remote application program CAP received from the server apparatus 16, and data received from the imaging apparatuses 13A-13C.

The memory 26 is a working memory with which the CPU 25 performs tasks. The CPU 25 loads the memory 26 with programs read from the storage device 27, and performs the tasks according to the programs to control various elements in the mobile terminal apparatus 10.

An antenna is included in the radio communication interface 28 for transmitting and receiving radio waves. There is a communication network 31 or intranet through which the radio communication interface 28 wirelessly communicates with the imaging apparatuses 13A-13C and the server apparatus 16.

The communication network 31 is a LAN (local area network) disposed in the hospital facility 12, for example, a mixture of a wired LAN and a wireless LAN. An example of the communication network 31 is constituted by a wireless access point and a network apparatus. The wireless access point is communicable with the radio communication interface 28 of the mobile terminal apparatus 10. The network apparatus is a switching hub, router and the like for wired connection to the wireless access point by use of a communication cable. For the purpose of communication between the mobile terminal apparatus 10 and any one of the imaging apparatuses 13A-13C and the server apparatus 16, the wireless access point is used in the communication network 31 for transmitting signals to or from the mobile terminal apparatus 10. The switching hub, router, communication cable and the like are used in the communication network 31 for transmitting signals to or from the imaging apparatuses 13A-13C.

The CT imaging apparatus 13A includes a controller 33A and a communication interface 34A which receives a control signal generated by the mobile terminal apparatus 10 and transmits a created image to the mobile terminal apparatus 10 by use of the communication network 31. The controller 33A controls the CT imaging apparatus 13A according to a control signal from the mobile terminal apparatus 10. Similarly, the X-ray imaging apparatus 13B includes a controller 33B and a communication interface 34B. The mammography apparatus 13C includes a controller 33C and a communication interface 34C.

Also, the server apparatus 16 includes a communication interface 45, a program distribution device 42 for the remote application program, and a program storage device 43 for the remote application program. The communication interface 45 communicates with the mobile terminal apparatus 10 by use of the communication network 31. The program distribution device 42 distributes the remote application program in response to a request from the mobile terminal apparatus 10. The program storage device 43 stores a plurality of remote application programs in accordance with plural modalities.

The program storage device 43 is a storage device such as a hard disk, and stores combinations of remote application programs CAP-A, CAP-B and CAP-C for the imaging apparatuses 13A-13C and modality IDs of A, B and C. The program distribution device 42, upon receiving a request from the mobile terminal apparatus 10 including the modality ID, searches and detects a remote application program CAP in the program storage device 43 according to the specified modality ID, and transmits the detected remote application program CAP to the mobile terminal apparatus 10 by use of the communication interface 45.

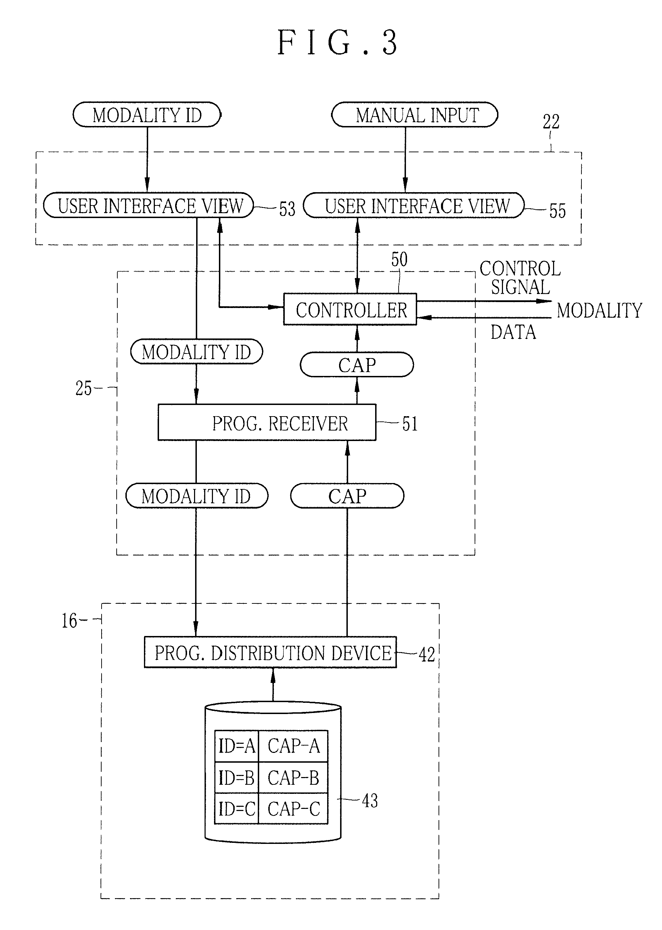

In Fig. 3, the CPU 25 functions by way of a controller 50 and a program receiver 51 upon startup of the basic program 30 in the mobile terminal apparatus 10. The controller 50 controls various elements in the mobile terminal apparatus 10. A user interface view 53 is displayed on the touchscreen device 22 according to the GUI (graphical user interface) in the basic program 30, so that the touchscreen device 22 receives manual inputs from an operator. The inputs include a modality ID of any one of the imaging apparatuses 13A, 13B and 13C. A keyboard image is displayed in the user interface view 53. The modality ID is input by manipulating keys in the keyboard image. The touchscreen device 22 as display device with the user interface view 53 operates as an information input device for inputting the modality ID in a manually operable structure. Display control of information displayed in the touchscreen device 22 is performed by the controller 50.

The modality ID (modality type code as modality information) input with the user interface view 53 is supplied to the program receiver 51. The program receiver 51 generates a request of transmission inclusive of the input modality ID, causes the radio communication interface 28 to send the request to the server apparatus 16, which acquires the remote application program CAP. The remote application program CAP is stored to the storage device 27. The user interface view 53 includes a portion of indicating a list of the remote application programs CAP stored in the storage device 27, and a start button for running the selected remote application program CAP.

Upon receiving a command signal for running the remote application program CAP from the user interface view 53, the controller 50 runs the selected remote application program CAP for a task of the remote management for the imaging apparatuses 13A-13C. The remote application program CAP includes a user interface view 55 of the GUI for each of the imaging apparatuses 13A-13C and various data of control signals. The controller 50 running the remote application program CAP drives the touchscreen device 22 to display the user interface view 55, and receives manual inputs of the operator through the user interface view 55. In short, the remote management is performed by running the remote application program CAP in the controller 50 for the imaging apparatuses 13A-13C.

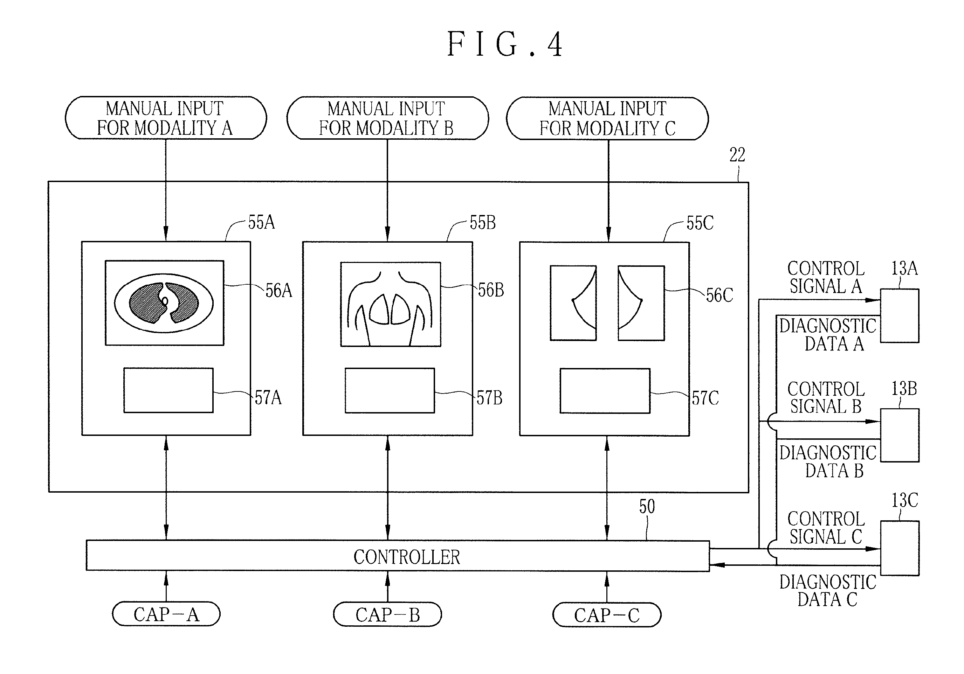

As illustrated in Fig. 4, the controller 50 drives the touchscreen device 22 to display a user interface view 55A according to the remote application program CAP-A upon running the same in association with the CT imaging apparatus 13A. The user interface view 55A has a viewing area for a CT image 56A and an input field 57A of a panel shape inclusive of buttons according to the GUI. The controller 50 receives an input of the CT imaging apparatus 13A through the input field 57A, and transmits a control signal A to the CT imaging apparatus 13A. Also, the controller 50 receives diagnostic data A including the CT image 56A formed by the CT imaging apparatus 13A, so that the diagnostic data A is displayed in the user interface view 55A.

Also, the controller 50 drives the touchscreen device 22 to display a user interface view 55B according to the remote application program CAP-B upon running the same in association with the X-ray imaging apparatus 13B. The user interface view 55B has a viewing area for an X-ray image 56B and an input field 57B of a panel shape. The controller 50 receives an input of the X-ray imaging apparatus 13B through the input field 57B, and transmits a control signal B to the X-ray imaging apparatus 13B. Also, the controller 50 receives diagnostic data B including the X-ray image 56B formed by the X-ray imaging apparatus 13B, so that the diagnostic data B is displayed in the user interface view 55B.

Also, the controller 50 drives the touchscreen device 22 to display a user interface view 55C according to the remote application program CAP-C upon running the same in association with the mammography apparatus 13C. The user interface view 55C has a viewing area for a mammographic image 56C and an input field 57C of a panel shape. The controller 50 receives an input of the mammography apparatus 13C through the input field 57C, and transmits a control signal C to the mammography apparatus 13C. Also, the controller 50 receives diagnostic data C including the mammographic image 56C formed by the mammography apparatus 13C, so that the diagnostic data C is displayed in the user interface view 55C.

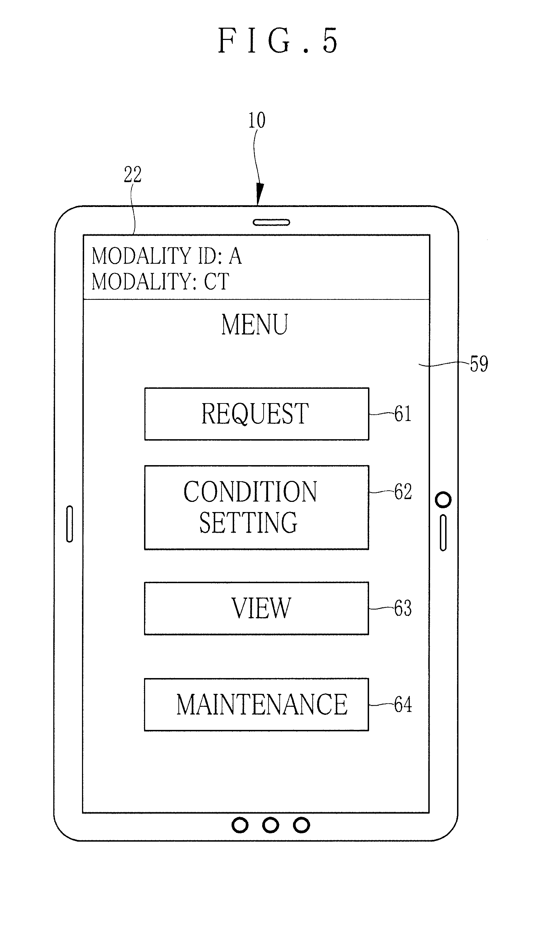

The user interface view 55 displays an image formed by each of the imaging apparatuses 13A-13C. The operator checks whether the image has been formed appropriately according to the user interface view 55. After the checking, the received image is transferred to and stored in a server apparatus (not shown). Details of the example of the user interface view 55 of the mobile terminal apparatus 10 will be hereinafter described by referring to the CT imaging apparatus 13A in Figs. 5-9.

In Fig. 5, a menu image 59 is displayed in the user interface view 55A of the CT imaging apparatus 13A, and has a request button 61, a condition setting button 62 (adjustment button), a view button 63 and a maintenance button 64.

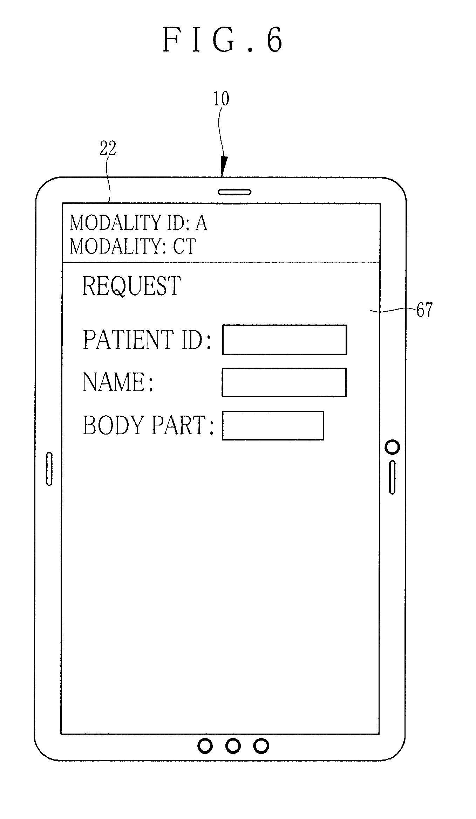

In case the request button 61 is selectively manipulated, a request area 67 is indicated as illustrated in Fig. 6. The request area 67 displays specific information of an imaging request, such as a case ID (identification data) of a patient, personal name, body part and the like. Examples of the body part are a head, chest, abdomen, whole body and the like. Data of the imaging request are stored in a Hospital Information System (HIS) or Radiology Information System (RIS) (not shown). The mobile terminal apparatus 10 acquires the imaging request through the communication network 31. Note that the imaging request can be input manually by an operator in the mobile terminal apparatus 10.

The imaging request is transmitted to the CT imaging apparatus 13A. This is for the purpose of recording the imaging request as auxiliary information of the image being formed. Also, it is possible in the mobile terminal apparatus 10 to record all of imaging requests for images formed by the CT imaging apparatus 13A The operator conditions the setting for imaging after viewing the request in the request area 67.

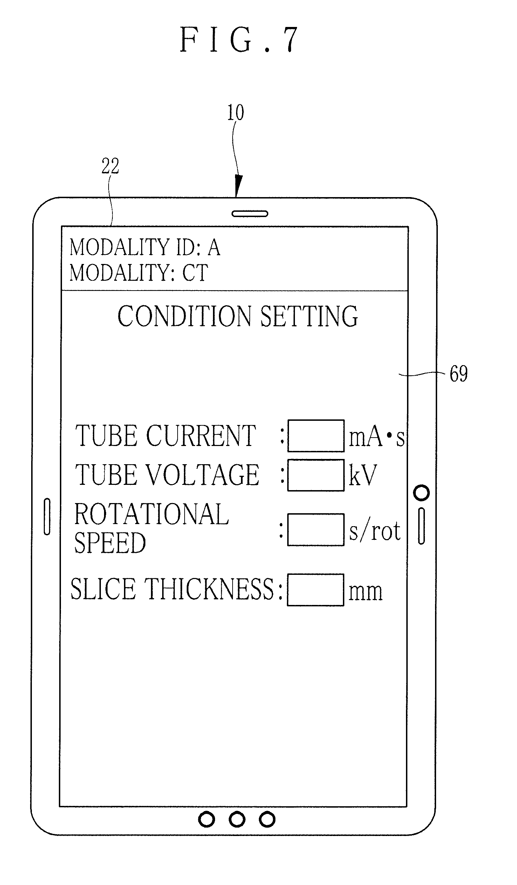

A condition setting area 69 (adjustment area) is displayed as illustrated in Fig. 7 upon selectively manipulating the condition setting button 62 in Fig. 5. Examples of parameter data in the imaging condition are a tube current for determining dose of X-rays, a tube voltage for determining quality (energy spectrum) of the X-rays, a rotational speed of an X-ray detector, and a collection slice thickness determined by a moving speed of the X-ray detector in an axial direction perpendicular to its rotational direction. Input fields are displayed in the condition setting area 69 in association with respectively the parameter data. The input imaging condition is transmitted to the CT imaging apparatus 13A. The CT imaging apparatus 13A creates an image by imaging according to the received imaging condition.

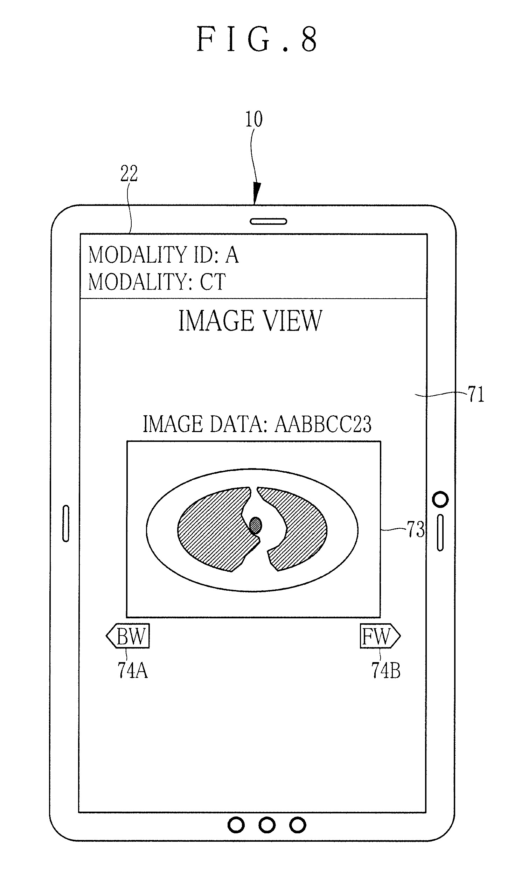

In case the view button 63 in Fig. 5 is selectively manipulated, a viewing area 71 or image display area is displayed as illustrated in Fig. 8. Imaging is carried out while the viewing area 71 is indicated. Before the imaging, there is no created body image 73 in the viewing area 71. After the imaging, the body image 73 is displayed. To this end, a common viewer without dependency to the modalities is used. According to the CT imaging apparatus 13A, a series of plural sectional images are obtained by the imaging. A forward button 74B and a backward button 74A are disposed under the body image 73 for serially changing over the image to be displayed. The forward and backward buttons 74A and 74B are used for stepping the body image 73 in respectively the forward and backward directions. It is possible in the viewing area 71 to check and correct the body image 73, attribute information associated with the body image 73, their sequence and the like before transmission of the body image 73 to a hospital facility. Also, images can be read from the server apparatus after the imaging, and can be displayed in the viewing area 71.

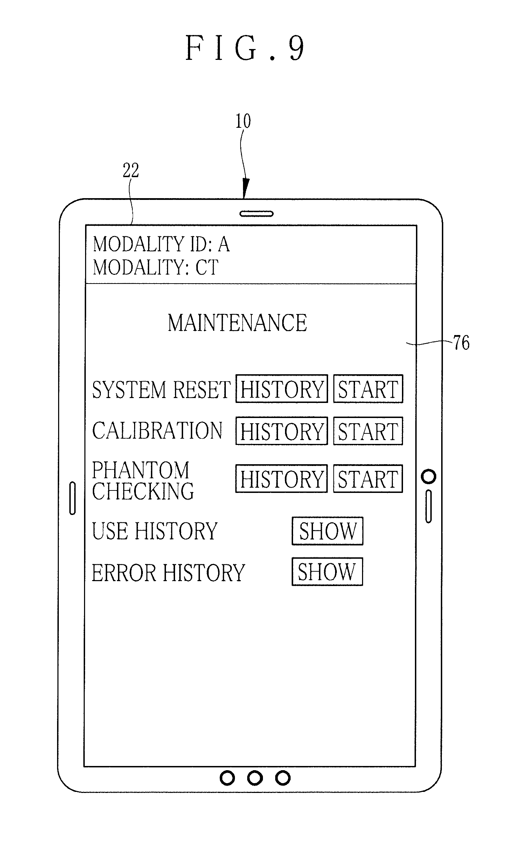

In case the maintenance button 64 in Fig. 5 is selectively manipulated, a maintenance area 76 is displayed as illustrated in Fig. 9. Examples of relevant data of the maintenance are command data of a system reset for initializing various statuses in the CT imaging apparatus, sensitivity correction and dark current correction of the X-ray imaging apparatus, command data of calibration of a rotational speed of a rotating mechanism in the X-ray imaging apparatus, calibration of a moving speed of a moving mechanism in the X-ray imaging apparatus, command data of phantom checking for evaluating performance of the CT imaging apparatus by use of a phantom or artificial body of a human body, and the like. Buttons are provided in the maintenance area 76 in combination with the relevant data, such as a start button for the maintenance and a history button for displaying information of maintenance history of the maintenance.

The information of the maintenance history is stored in the CT imaging apparatus 13A. In case the history button is manipulated, a request signal for the maintenance history is transmitted to the CT imaging apparatus 13A, which responsively sends the maintenance history to the mobile terminal apparatus 10. Also, various buttons are disposed in the maintenance area 76, including a history button for displaying a use history of the number, date and the like of events of the imaging, and an error button for displaying a date of occurrence of an error. The use history and the error history are stored in the CT imaging apparatus 13A together with the maintenance history, read from the CT imaging apparatus 13A in response to a command signal from the mobile terminal apparatus 10, and displayed on the touchscreen device 22. Note that it is possible to check other information through the maintenance area 76, the information including manual information, release information, a name of service patch used so far, and the like.

The remote management of the imaging apparatuses 13B and 13C is similar to the CT imaging apparatus 13A of which the user interface view 55 has been described. Functions for condition setting and maintenance are provided in the remote application programs CAP-B and CAP-C for the imaging apparatuses 13B and 13C. A condition setting area and maintenance area are indicated for each of those. The common viewer without dependency to the modalities is used for displaying images. A function for image display is provided in the remote management for the imaging apparatuses 13B and 13C. A viewing area for the image display is indicated for each of those. In the viewing area for any one of the imaging apparatuses 13B and 13C, it is possible to display, change over and visually check an image by manipulation similar to that of the CT imaging apparatus 13A.

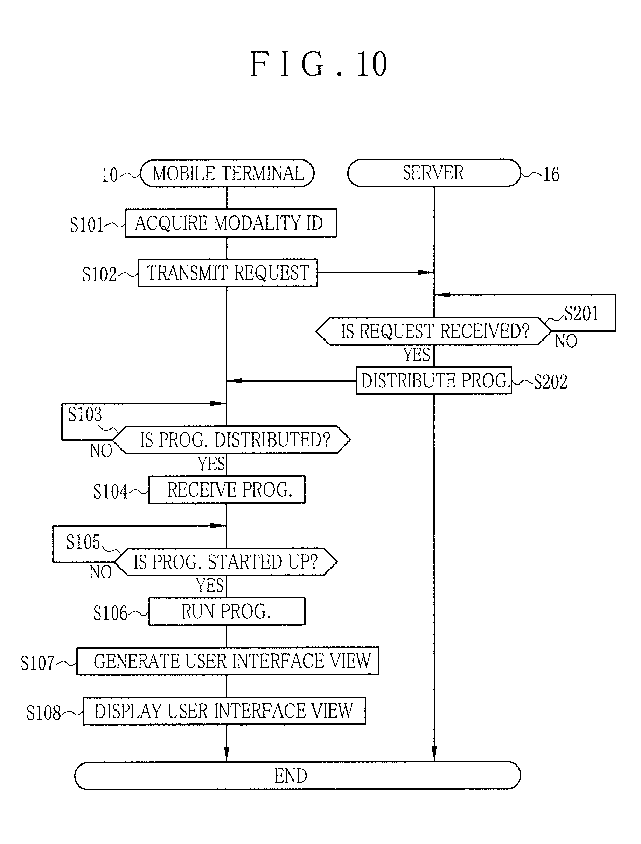

The operation of the mobile terminal apparatus 10 of the invention is hereinafter described by referring to the flow chart of Fig. 10.

To control any of the imaging apparatuses 13A-13C by use of the mobile terminal apparatus 10, at first an operator inputs a modality ID (modality type code as modality information) by use of the user interface view 53 in the touchscreen device 22 in association with a target apparatus among the modalities. See Fig. 3. Thus, the program receiver 51 of the mobile terminal apparatus 10 acquires the modality ID in the step S101.

The program receiver 51 transmits the request to the server apparatus 16 inclusive of the acquired modality ID (step S102). The server apparatus 16 always monitors the state as to whether the request has been received or not (step S201). Assuming that the request has been received (yes in the step S201), then the server apparatus 16 transmits the specified remote application program CAP (program information) to the mobile terminal apparatus 10 (step S202).

The mobile terminal apparatus 10 monitors occurrence of transmission of the remote application program CAP from the server apparatus 16 in the step S103 after transmitting the request. In response to transmitting the remote application program CAP (yes in the step S103), the remote application program CAP is received in the step S104. After this, the mobile terminal apparatus 10 monitors occurrence of an input of running the remote application program CAP from the operator in the step S105. The mobile terminal apparatus 10 in response to the input of running the remote application program CAP (yes in the step S105) starts up the remote application program CAP in the step S106. The mobile terminal apparatus 10 generates the user interface view 55 in the step S107 according to the remote application program CAP, to display the user interface view 55 in the touchscreen device 22 in the step S108.

While the user interface view 55 is displayed according to the remote application program CAP, the mobile terminal apparatus 10 functions as a remote terminal for a modality according to the remote application program CAP. The operator inputs a signal with the user interface view 55 for the modality. He or she confirms a request for imaging and sets an imaging condition by viewing the user interface view 55, and carries out imaging. In case an image is formed by the modality, the image is transmitted from the modality to the mobile terminal apparatus 10. The mobile terminal apparatus 10 displays the image in the user interface view 55. He or she visually confirms exactitude of the imaging by checking the image in the user interface view 55. The checked image from the mobile terminal apparatus 10 is transmitted to and stored in the server apparatus (not shown).

For the maintenance, a control signal is transmitted through the maintenance area 76 to the modality for command according to the relevant data of the maintenance.

To use another modality, the steps S101 to S108 are repeated.

As described heretofore, it is possible with the mobile terminal apparatus 10 of the invention remotely to manage or control the imaging apparatuses 13A-13C of different methods commonly inside the hospital facility 12. Also, the mobile terminal apparatus 10 acquires the remote application program CAP by accessing the server apparatus 16 storing the remote application programs CAP according to the imaging apparatuses 13A-13C, and runs the received remote application program CAP to perform the remote management according to the remote application program CAP. Thus, the remote application program CAP can be updated easily by operation of an operator as required. Assuming that the version of the remote application program CAP is updated or a new modality is introduced, an updated remote application program CAP or one for the new modality can be uploaded, so that the mobile terminal apparatus 10 accesses the server apparatus 16 to install the remote application program CAP.

It is possible considerably to reduce time, labor cost and the like according to the invention in comparison with a conventional method in which the mobile terminal apparatus 10 used by an operator is collectively withdrawn and updated for a new remote application program CAP.

Also, the handlability of the mobile terminal apparatus 10 in use can be high because the maintenance function is included in the functions of the remote management with the remote application program CAP in addition to the condition setting function.

Note that in the above embodiments, the remote application program CAP is stored in the storage device 27 after being transmitted from the server apparatus 16, and can be reused at the time of next startup without retransmission from the server apparatus 16, because stored in the storage device 27. There may occur a problem in the shortage in the capacity of the storage device 27 assuming that a great number of the remote application programs CAP are stored. In view of this, it is possible automatically to delete one or more of the remote application programs CAP of which frequency of the use is relatively low, to enlarge an unused memory space of the storage device 27.

In the above embodiment, the modality ID in the mobile terminal apparatus 10 is acquired by a manual input with the touchscreen device 22 as a display device. However, the radio communication interface 28 can be an information input device for inputting a modality ID.

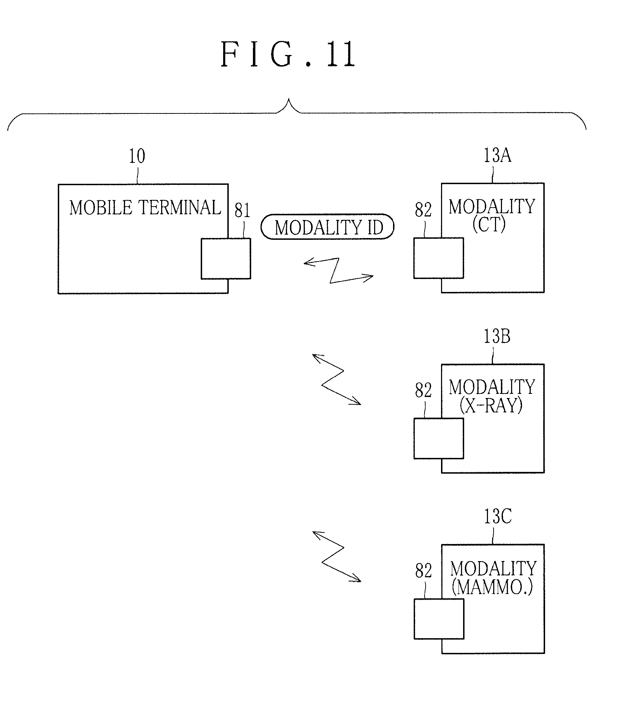

Another preferred embodiment is illustrated in Fig. 11, in which a short range radio communication interface 81 is provided in the mobile terminal apparatus 10 in a separate manner from the radio communication interface 28. The short range radio communication interface 81 is used as an information input device.

Also, a short range radio communication interface 82 is provided in each of the imaging apparatuses 13A-13C to correspond to the short range radio communication interface 81. Examples of each of the short range radio communication interfaces 81 and 82 are an interface of an infrared communication standard for use with infrared rays, an interface of a Bluetooth standard (trade name) for use with radio waves of 2.4 GHz, and the like for communication within a range from several centimeters to several meters. Also, the short range radio communication interface can be an RFID tag (radio frequency identification tag) and the like.

It is unnecessary to input the modality ID manually by use of the short range radio communication interface 81. This is effective in reducing the labor cost in the manipulation and preventing errors in the input. Furthermore, a use of an RFID tag (radio frequency identification tag) is advantageous to simplify the manipulation, because the modality ID can be input only by setting the mobile terminal apparatus 10 near to the imaging apparatuses 13A-13C.

It is possible for the short range radio communication interface 81 to emit a search signal always to search a modality located near to the mobile terminal apparatus 10, so that a modality ID of the searched modality can be automatically transmitted upon receiving the search signal.

Furthermore, it is possible to acquire the modality ID both by the short range radio communication interface 81 and by a manual input.

The user interface view of the above embodiment is only one example. Various other user interface views can be used for the similar performance. In the above embodiments, the maintenance function is included in the remote management. However, the maintenance function may not be provided.

The modalities are the CT imaging apparatus, X-ray apparatus and mammography apparatus according to the above embodiments. However, their number and imaging methods can be changed. Examples of the modalities are an MRI apparatus (magnetic resonance imaging apparatus), endoscope apparatus, ultrasonic imaging apparatus and the like. Also, a combination of two or more of those can be used. Furthermore, examples of plural modalities can be apparatuses of two of more different models or two or more different dates of production even for the CT imaging apparatus, X-ray apparatus or the like.

In the above embodiments, the mobile terminal apparatus 10 is used for communication access to the modalities of different imaging methods. However, it is possible with the mobile terminal apparatus 10 to perform the communication access to plural CT imaging apparatuses or the like as a single modality. The common remote application program CAP can be used for the use of the apparatuses of the single modality. Assuming that the plural apparatuses of the single modality must be distinguished from one another, it is possible to acquire plural sets of the modality ID. A control signal is output by assigning the acquired modality ID to prevent crosstalk between control signals.

Note that the mobile terminal apparatus of the invention sends and receives a signal in connection with the modalities according to the remote management. However, it is possible only to send a signal to the modalities to control or drive the modalities. Also, it is possible only to receive a signal from the modalities to monitor or check the modalities.

Note that the communication access according to the invention can be used in various conditions of a pair of locations. For example, the mobile terminal apparatus 10 can be located considerably near to the modality but for access thereto in a non-contact manner. Also, the mobile terminal apparatus 10 can be located at a long distant from the modality, for example, in the course of a personal travel of a doctor or user with the mobile terminal apparatus 10, and can manage the modality in the remote operation.

In the above embodiments, the mobile terminal apparatus is a cellular telephone or smart phone. However, the mobile terminal apparatus can be a tablet or notebook computer, laptop computer, personal digital assistant (PDA), mobile Internet device (MID), digital broadcast receiver or the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A mobile terminal apparatus having a radio communication interface (28) for radio communication, comprising:
a display device (22);
a program receiver (51) for receiving a particular program (CAP) for a particular modality (13A-13C) of interest from a program storage apparatus (16) storing plural programs in association with respectively modalities;
a controller (50) for functioning according to said particular program, for driving said display device to display a user interface view (53, 55, 55A-55C, 59), and for causing said radio communication interface to perform communication access to said particular modality.

2. A mobile terminal apparatus as defined in claim 1, wherein said controller transmits a signal to and receives a signal from said particular modality by said communication access.

3. A mobile terminal apparatus as defined in claim 1 or 2, further comprising an information input device for inputting modality information for recognition of each of said modalities;
wherein said program receiver transmits said input modality information to said program storage apparatus, and acquires said particular program from said program storage apparatus.

4. A mobile terminal apparatus as defined in claim 3, wherein said information input device is externally operated to input said modality information.

5. A mobile terminal apparatus as defined in claim 3 or 4, wherein said information input device receives said modality information transmitted from said particular modality with said radio communication interface.

6. A mobile terminal apparatus as defined in claim 3, 4 or 5, wherein said information input device is a short range radio communication interface discrete from said radio communication interface for receiving said modality information from said particular modality.

7. A mobile terminal apparatus as defined in any one of claims 1 to 6, wherein said controller carries out display control for displaying received data on said display device upon reception from said particular modality.

8. A mobile terminal apparatus as defined in claim 7, wherein said received data is an image.

9. A mobile terminal apparatus as defined in any one of claims 1 to 8, wherein said program receiver accesses said program storage apparatus by use of said radio communication interface.

10. A mobile terminal apparatus as defined in any one of claims 1 to 9, wherein said modality is at least one of a CT imaging apparatus, X-ray imaging apparatus and mammography apparatus.

11. A mobile terminal apparatus as defined in any one of claims 1 to 10, wherein said user interface view includes a request area for requesting said modality to create an image.

12. A mobile terminal apparatus as defined in claim 11, wherein said request area is used for inputting at least one of name data of a body, identification data of said body, and body part data in said body.

13. A mobile terminal apparatus as defined in any one of claims 1 to 12, wherein said user interface view includes a condition setting area for inputting parameter data of condition setting of said particular modality.

14. A mobile terminal apparatus as defined in any one of claims 1 to 13, wherein said user interface view includes a maintenance area for transmitting or receiving relevant data of maintenance of said particular modality.

15. A mobile terminal apparatus as defined in claim 14, wherein said relevant data is at least one of command data of reset, command data of calibration, use history data and error history data of the particular modality.
